(19) Europäisches Patentamt
European Patent Office
Office européen des brevets

(11) **EP 0 944 440 B1**

(12) **FASCICULE DE BREVET EUROPEEN**

(45) Date de publication et mention
de la délivrance du brevet:
**23.06.2004 Bulletin 2004/26**

(51) Int Cl.⁷: **B05C 17/01**, A61C 5/06,
A61M 5/24

(21) Numéro de dépôt: **97952984.9**

(22) Date de dépôt: **23.12.1997**

(86) Numéro de dépôt international:
**PCT/FR1997/002391**

(87) Numéro de publication internationale:
**WO 1998/028090 (02.07.1998 Gazette 1998/26)**

(54) **DISPOSITIF DE DISTRIBUTION D'UNE MATIERE EXTRUDABLE TELLE QU'UNE PATE A USAGE DENTAIRE**

VORRICHTUNG ZUR AUSGABE VON EXTRUDIERBAREN MATERIAL WIE DENTALMASSEN

DEVICE FOR DISPENSING AN EXTRUSIBLE SUBSTANCE SUCH AS A PASTE FOR DENTAL USE

(84) Etats contractants désignés:
**CH DE ES FR GB IT LI**

(30) Priorité: **23.12.1996 FR 9615847**

(43) Date de publication de la demande:
**29.09.1999 Bulletin 1999/39**

(73) Titulaire: **LESAGE, Patrick**
**F-35400 Saint-Malo (FR)**

(72) Inventeur: **LESAGE, Patrick**
**F-35400 Saint-Malo (FR)**

(74) Mandataire: **Hubert, Philippe**
**Cabinet Beau de Loménie**
**158, rue de l'Université**
**75340 Paris Cédex 07 (FR)**

(56) Documents cités:
| | |
|---|---|
| **BE-A- 515 066** | **FR-A- 641 809** |
| **FR-A- 963 422** | **FR-A- 1 320 820** |
| **FR-A- 2 245 382** | **US-A- 1 574 579** |
| **US-A- 1 818 670** | |

## Description

La présente invention a pour objet un dispositif de distribution d'une matière extrudable.

L'invention trouve notamment application dans le domaine dentaire, pour la distribution de produits pâteux tels que par exemple des matériaux pour la prise d'empreintes, des cires pour l'obturation canalaire, des résines composites ou des ciments pour les traitements de restauration des dents ou encore des matériaux d'insertion tels que celui décrit dans le document WO-A-90/15587, utilisé pour élargir le sillon gingival.

D'une façon plus générale, la présente invention peut être utilisée dans d'autres domaines pour l'extrusion de matières variées telles que des colles, des ciments, des cires ou des élastomères.

Par "matière extrudable" on entend désigner ici toute matière qui, à la différence des matières liquides, ne s'écoule pas sous le simple effet de la gravité. De telles matières peuvent présenter des viscosités variées. A titre d'exemple, la viscosité du matériau d'insertion décrit dans le brevet français N°8907812 auquel l'invention s'applique plus particulièrement, est comprise entre environ 13.000 et environ 30.000 Pa.s.

De nombreux dispositifs de distribution d'une matière extrudable ont été proposés dans l'état de la technique et notamment dans le domaine dentaire.

De tels dispositifs comprennent généralement un réservoir destiné à contenir ladite matière à extruder et à coopérer avec des moyens formant piston coulissant dans ledit réservoir et aptes à permettre le transfert de ladite matière dans un embout prolongeant ledit réservoir.

On distingue dans l'état de la technique deux grandes catégories de dispositifs de distribution du type précité :

- ceux dans lesquels le réservoir et l'embout sont constitués d'une pièce unique ;
- ceux dans lesquels le réservoir et l'embout sont constitués de deux éléments distincts.

La présente invention concerne plus particulièrement un dispositif appartenant à cette deuxième catégorie.

Le principal problème à résoudre, pour la conception d'un dispositif appartenant à cette deuxième catégorie est bien entendu celui de l'étanchéité entre l'embout et le réservoir, en particulier au cours de la phase de refoulement de la matière.

Pour résoudre ce problème technique, les dispositifs connus jusqu'à ce jour font appel à des fixations mécaniques plus ou moins élaborées entre l'embout et le réservoir, comme par exemple une fixation par vissage ou une fixation du type à baïonnette.

De tels dispositifs ont notamment été décrits dans les brevets US 3.121.516 et US 3.767.085.

S'ils apportent une solution satisfaisante au problème de l'étanchéité entre l'embout et le réservoir, ces dispositifs connus sont souvent fragiles, présentent une forme complexe facilement encrassée par le produit extrudé et difficile à nettoyer. Ils sont d'une mise en oeuvre relativement peu aisée lors du changement de l'embout ou du réservoir.

On connaît également dans l'état de la technique, et notamment dans le domaine dentaire, des dispositifs destinés à l'injection de liquide, notamment par voie hypodermique, comportant un réservoir et un embout séparable.

De tels dispositifs ont par exemple été décrits dans les documents BE 515.066, FR 2.245.382, FR 1.320.820, US 1.818.670.

Dans ces dispositifs connus, le réservoir contenant le liquide à injecter est généralement constitué d'une ampoule de verre, et comporte un orifice obturé par un bouchon, éventuellement perforable, réalisé en une matière élastomérique.

Le bouchon comporte un orifice central dans lequel ledit embout peut être emmanché à force avec frottement.

L'étanchéité entre l'embout et le réservoir contenant le liquide à injecter est par conséquent assurée par la coopération de deux surfaces cylindriques fermement liées entre elles par un serrage radial, l'une des surfaces étant en outre déformable élastiquement.

Outre, qu'ils ne sont pas destinés à la distribution de matières extrudables, ces dispositifs connus présentent l'inconvénient de conduire à une fixation mécanique de l'embout sur le réservoir, l'embout n'étant séparable dudit réservoir que sous l'effet d'une force extérieure relativement importante.

En outre, le réservoir étant constitué de verre, il est particulièrement fragile et ne pourrait en aucun cas convenir pour la distribution d'une matière extrudable.

Un dispositif d'une conception voisine de celle décrite dans les documents précités est révélé par le document FR 641 819.

Dans ce dispositif, l'ampoule contenant le médicament à injecter présente une extrémité conique munie d'un collier élastique 24. Une aiguille hypodermique 42 comprenant un corps 43 muni d'une cavité 47 est montée sur la seringue 10.

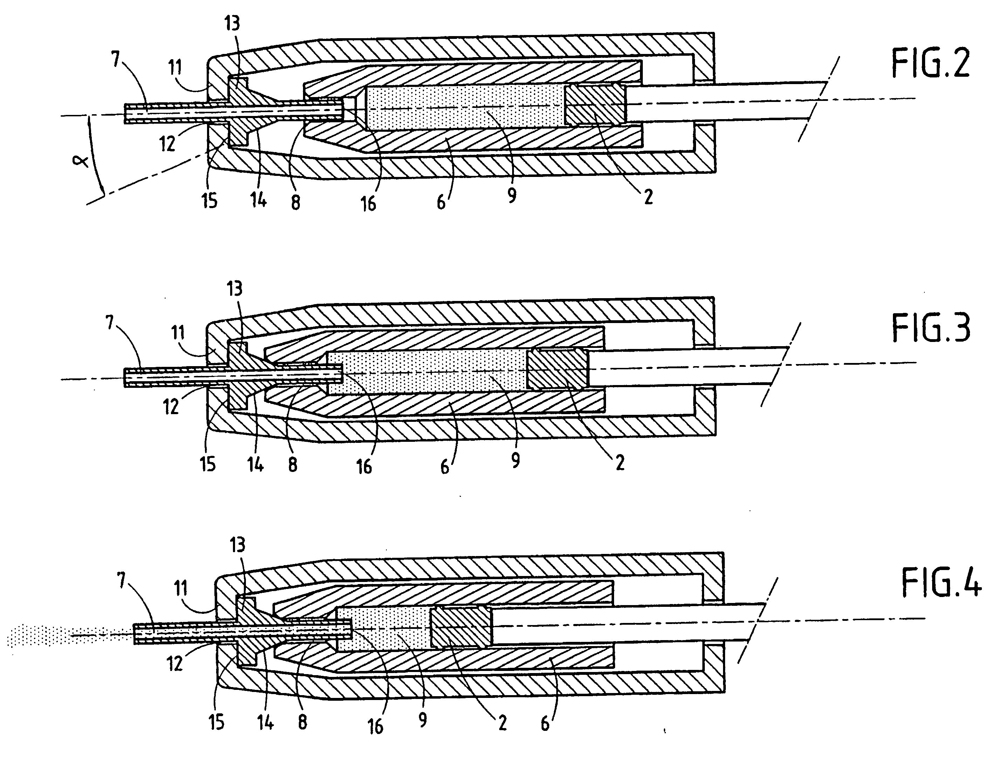

Le collier élastique 24 prend appui dans la cavité conique 47 du corps 43 de l'aiguille hypodermique comme le montre la figure 4.

Ainsi, l'étanchéité de ce dispositif connu est assurée par la coopération de deux surfaces tronconiques avec interposition d'un joint élastique.

Ce dispositif présente donc sensiblement les mêmes inconvénients qu'énoncés précédemment.

Dans ces conditions, la présente invention a pour but de résoudre le problème technique consistant en la fourniture d'un dispositif de distribution d'une matière extrudable d'une nouvelle conception, garantissant une parfaite étanchéité entre le réservoir et l'embout pendant la phase de refoulement de la matière, sans faire appel à des moyens de fixation mécanique, tout en permettant une séparation très aisée du réservoir et de l'embout, après ladite phase de refoulement.

La présente invention a encore pour but de résoudre ce problème technique par la fourniture d'un dispositif qui soit facile à mettre en oeuvre et d'un coût relativement modéré.

La solution conforme à la présente invention pour résoudre ce problème technique consiste en un dispositif de distribution d'une matière extrudable comprenant :

- un réservoir destiné à contenir la matière et comportant au moins un orifice ;
- un embout tubulaire séparable communiquant, en position d'utilisation, avec ledit réservoir par ledit orifice;
- un piston apte à coulisser, en position d'utilisation, dans ledit réservoir et à exercer sur ladite matière une force suffisante pour la refouler à l'extérieur via l'embout ;
- une embase portant des moyens de commande en déplacement dudit piston et comportant un élément formant plaque disposé dans un plan sensiblement orthogonal à la direction de déplacement dudit piston, ladite plaque présentant un passage apte à recevoir ledit embout et disposé en regard de l'orifice dudit réservoir;
- une pièce d'étanchéité disposée entre ledit élément formant plaque 11 et ledit réservoir 6 et présentant un orifice prolongeant le passage de ladite plaque ;
- des moyens pour réaliser une liaison étanche entre ledit embout et ladite pièce d'étanchéité, au moins sous l'effet du déplacement dudit réservoir vers ladite plaque ; et
- des moyens pour immobiliser ladite pièce d'étanchéité par rapport à ladite plaque, au moins sous l'effet du déplacement dudit réservoir vers ladite plaque,

caractérisé en ce que ladite pièce d'étanchéité présente une face d'étanchéité tournée vers ledit réservoir; ladite face d'étanchéité et la paroi définissant l'orifice étant conformées pour coopérer par contact d'appui sensiblement selon une ligne en étant ainsi aptes :

- d'une part, à coopérer, par contact d'appui, sensiblement sans frottement, sous l'effet du déplacement dudit réservoir vers ladite plaque en réponse au déplacement dudit piston dans ledit réservoir, pour réaliser ainsi une liaison étanche ; et
- d'autre part, à être automatiquement séparées l'une de l'autre sous l'effet du déplacement dudit réservoir en direction opposée à ladite plaque.

Ainsi, l'originalité du dispositif conforme à la présente invention réside dans le fait que c'est la force développée par le piston pour l'extrusion de la matière qui assure la mise en contact et le maintien en contact étanche entre l'embout séparable et le réservoir. Plus cette force est importante et plus l'étanchéité est forte. Il n'y a donc aucune limite quant à la viscosité de la matière à extruder.

En outre, en raison de la conformation particulière de la face d'étanchéité et de la paroi de l'orifice du réservoir, l'embout et ledit réservoir sont automatiquement séparés dès lors que ces faces ne sont pas maintenues en contact d'appui l'une contre l'autre.

L'embout étant séparable du réservoir, son changement est donc extrêmement aisé, dans la mesure où il n'y a pas de fixation mécanique et donc aucune opération de déverrouillage préalable au démontage de l'embout.

Contrairement aux dispositifs connus de l'état de la technique, il n'existe pas de serrage radial entre l'embout et le réservoir, le contact d'appui entre la face d'étanchéité et la paroi de l'orifice du réservoir ayant une composante axiale et non pas une composante essentiellement radiale.

En outre, contrairement aux dispositifs connus de l'état de la technique, l'étanchéité du dispositif conforme à l'invention est réalisée essentiellement le long d'une ligne et non pas par coopération de deux surfaces entre elles.

Selon une caractéristique particulière du dispositif conforme à l'invention, ladite pièce d'étanchéité est portée de façon périphérique et coaxiale par ledit embout.

Selon un mode de réalisation actuellement préféré de l'invention, la pièce d'étanchéité précitée présente une première face sensiblement tronconique formant ladite face d'étanchéité et une deuxième face sensiblement plane apte à venir en appui contre ladite plaque formant ainsi les moyens précités pour immobiliser ladite pièce d'étanchéité par rapport à ladite plaque.

L'invention sera mieux comprise, et d'autres buts, caractéristiques et avantages de celle-ci apparaîtront plus clairement à la lecture de la description explicative qui va suivre faite en référence aux dessins annexés donnés uniquement à titre non limitatif et illustrant un mode de réalisation actuellement préféré de l'invention, et dans lesquels :

- la figure 1 est une vue en coupe transversale d'un dispositif de distribution d'une matière extrudable conforme à la présente invention montrant l'embout et le réservoir au début de leur phase de positionnement ;
- la figure 2 est une vue en coupe transversale de la partie avant du dispositif représenté à la figure 1 montrant l'embout et le réservoir à l'issue de leur phase initiale de positionnement ;
- la figure 3 est une vue semblable à la figure 2 montrant l'embout et le réservoir au cours de leur phase de rapprochement et de mise en contact ;

- la figure 4 est une vue semblable aux figures 2 et 3 montrant l'embout et le réservoir au cours de la phase de refoulement de la matière ; et
- la figure 5 est une vue semblable à la figure 2 illustrant le principe sur lequel la présente invention repose.

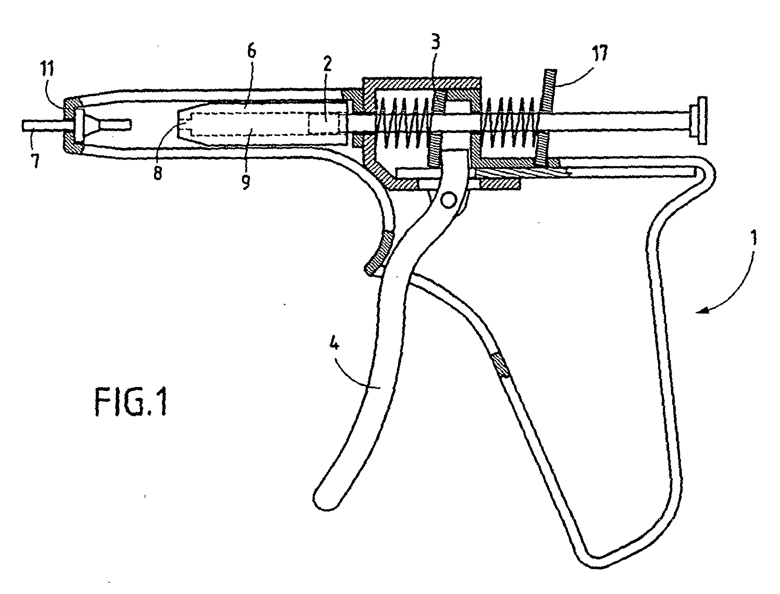

On a donc représenté à la figure 1 un dispositif de distribution d'une matière extrudable conforme à la présente invention.

Ce dispositif comporte une embase généralement représentée par le chiffre de référence 1 portant des moyens de commande en déplacement d'un piston 2 dont la tige est portée par ladite embase.

Ces moyens de commande en déplacement du piston peuvent être de conformations variées et comportent dans l'exemple représenté un élément formant plaque 3 susceptible d'être actionné par une poignée 4 conformée pour transmettre, en la multipliant par effet de levier, la force développée par l'utilisateur entre les doigts et la paume de la main.

L'embase 1 est destinée à loger, comme il sera expliqué ci-après, un réservoir 6 destiné à contenir la matière à extruder et un embout séparable 7, de forme généralement tubulaire, disposé dans le prolongement de ce dernier.

Le réservoir 6 comporte dans sa partie avant (ou partie mésiale) une face comportant un orifice 8 défini par une paroi sensiblement cylindrique et débouchant dans une chambre 9 de forme sensiblement cylindrique contenant la matière à extruder, ouverte sur sa face opposée à celle comportant l'orifice 8.

Le piston 2 est adapté pour coulisser dans la chambre 9 du réservoir 6 de façon à exercer sur la matière une force suffisante pour la refouler vers l'extérieur au travers de l'orifice 8.

L'embase 1 est solidaire d'un élément formant plaque 11 disposé dans un plan sensiblement orthogonal à la direction de déplacement du piston 2, ladite plaque présentant un passage 12 apte à recevoir l'embout 7 et disposé en regard de l'orifice 8 de réservoir.

Dans l'exemple représenté, l'élément formant plaque 11 fait partie intégrante de l'embase 1 et en constitue l'extrémité avant.

D'une façon générale, un dispositif de distribution conforme à la présente invention comprend en outre une pièce d'étanchéité spécifiquement conformée pour raccorder l'embout 7 au réservoir 6 sans fixation et de manière étanche pendant la phase de refoulement de ladite matière.

A cette fin, la pièce d'étanchéité présente généralement d'une part un orifice prolongeant le passage 12 de la plaque 11, et d'autre part une face d'étanchéité tournée vers le réservoir 6 et apte à coopérer, notamment par un contact d'appui sensiblement sans frottement, et sans serrage radial, avec l'orifice 8 en réalisant une étanchéité entre elle-même et la paroi dudit orifice sous l'effet du déplacement du réservoir 6 vers la plaque 11 en réponse au déplacement du piston 2 dans le réservoir 6.

De plus, cette face d'étanchéité est également conformée pour se séparer automatiquement de la paroi de l'orifice 8 dès lors qu'elle n'est plus maintenue en contact avec cette dernière, c'est-à-dire sous l'effet du déplacement du réservoir en direction opposée à la plaque 11 en réponse au retrait dudit piston.

De plus, le dispositif de distribution conforme à l'invention comporte généralement des moyens pour réaliser une liaison étanche entre l'embout 7 et la pièce d'étanchéité ainsi que des moyens pour immobiliser ladite pièce d'étanchéité par rapport à la plaque 11, qui seront décrits plus en détail par la suite.

Selon un mode de réalisation actuellement préféré de l'invention, la pièce d'étanchéité généralement représentée par le chiffre de référence 13 est portée de façon périphérique et coaxiale par l'embout 7.

La pièce d'étanchéité 13 sera généralement fixée de façon irréversible à l'embout 7, par exemple par soudure ou collage, ou bien encore ces deux éléments peuvent former une pièce monobloc comme dans l'exemple représenté.

Bien entendu, la pièce d'étanchéité 13 peut être également fixée de façon réversible à l'embout 7, par exemple par vissage ou par ajustage à frottement dur.

Dans tous les cas, il conviendra de s'assurer de la parfaite étanchéité entre l'embout 7 et la pièce d'étanchéité 13.

Selon un mode de réalisation non représenté de l'invention, la pièce d'étanchéité précitée fait partie intégrante de la plaque 11 et fait saillie hors de celle-ci.

La pièce d'étanchéité 13 peut être disposée, de telle sorte que l'extrémité proximale 16 de l'embout débouche à l'intérieur de la chambre 9 du réservoir 6 comme dans l'exemple représenté.

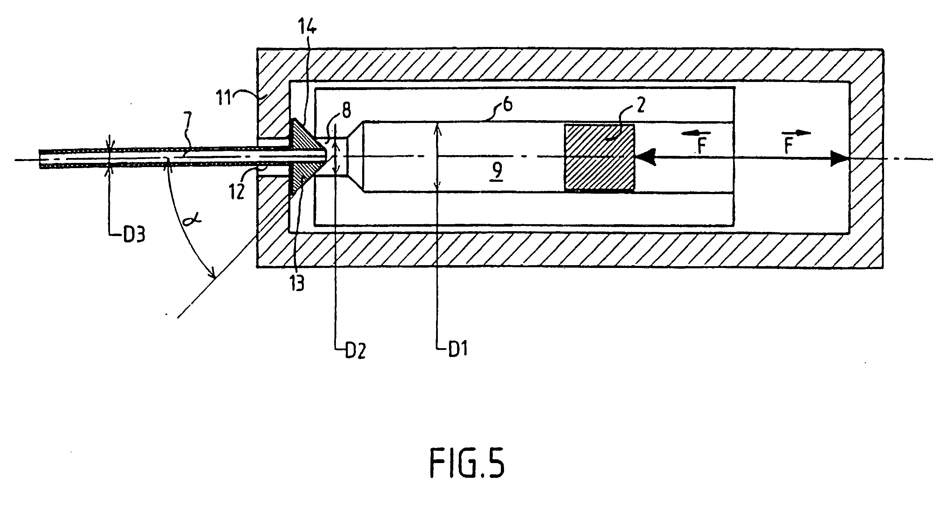

Cependant, le principe général de l'invention (figure 5) garantit une parfaite étanchéité entre la pièce d'étanchéité 13 et l'orifice 8 y compris dans le cas où l'extrémité proximale de l'embout ne débouche pas à l'intérieur de la chambre 9 du réservoir 6.

Dans ce cas, la pièce d'étanchéité 13 sera disposée à l'extrémité de l'embout 7, comme représenté à la figure 5.

Selon une caractéristique particulière de l'invention, la pièce d'étanchéité 13 présente une première face 14 de forme sensiblement tronconique formant la face d'étanchéité précitée et une deuxième face sensiblement plane 15, opposée à ladite première face et destinée à venir en appui contre la plaque 11 et à immobiliser la pièce d'étanchéité 13 par rapport à la plaque 11, au moins sous l'effet du déplacement du réservoir 6 vers la plaque 11 en réponse au déplacement du piston 2 dans le réservoir 6.

La première face 14 peut également être de forme sensiblement hémisphérique.

Avantageusement, la paroi définissant l'orifice 8 est sensiblement cylindrique.

Il est important, selon l'invention, que la face d'étanchéité 14 et la paroi définissant l'orifice 8 viennent en contact l'une avec l'autre sous l'effet du déplacement du réservoir 6 vers la plaque 11 selon une ligne (intersection d'une surface tronconique ou hémisphérique et d'une surface cylindrique).

Un tel contact est réalisé, comme on le comprend, sensiblement sans frottement, et sans serrage radial à la différence des dispositifs connus de l'état de la technique.

Selon une autre caractéristique particulière de l'invention, la face d'étanchéité de la pièce d'étanchéité et la paroi définissant l'orifice 8 du réservoir 6 sont réalisées dans des matières aptes à permettre une déformation relative des parties en contact, au moins sous l'effet du déplacement du réservoir 6 vers la plaque 11 en réponse au déplacement du piston 2 dans le réservoir 6, en garantissant ainsi une parfaite étanchéité.

Le choix de ces matières dépend essentiellement des contraintes mécaniques qui dépendent elles-mêmes directement de la viscosité du produit.

A titre d'exemple, la face d'étanchéité de la pièce d'étanchéité peut être réalisée en une matière plastique telle que par exemple du polypropylène, tandis que la paroi de l'orifice 8 du réservoir 6 peut être réalisée en métal.

La face d'étanchéité 14 et la paroi de l'orifice 8 peuvent également être réalisées toutes deux en une matière plastique telle que par exemple du polypropylène.

L'angle $\alpha$ définissant la surface tronconique de la face d'étanchéité 14 peut varier dans de larges limites et dépend, comme on le comprend, de la viscosité du produit à extruder.

Cet angle sera généralement compris entre 5 et 85° et sera de préférence voisin de 20° pour un produit de viscosité comprise entre 13 000 et 30 000 Pa.s à 20°C.

La paroi définissant l'orifice 8 du réservoir 6 peut être cylindrique, comme indiqué précédemment, ou peut encore former un bord chanfreiné.

Dans ce dernier cas, l'angle formant le bord chanfreiné sera avantageusement différent de l'angle $\alpha$ définissant la surface tronconique de la face d'étanchéité 14, de façon à ce que le contact entre la face d'étanchéité 14 et la paroi de l'orifice 8 s'établisse, comme mentionné précédemment, sensiblement selon une ligne.

Les diamètres D1 de la chambre 9 du réservoir 6, D2 de l'orifice 8 du réservoir 6 et D3 de l'embout 7 seront avantageusement choisis pour assurer une bonne distribution de la matière à extruder sans nuire à l'étanchéité. Ces diamètres pourront être facilement déterminés par l'homme de métier.

Dans le mode de réalisation actuellement préféré de l'invention, ces diamètres peuvent être choisis dans les rapports suivants :

$$D_1 = 2D_2 \ ;$$

$$3D_3 = 2D_2.$$

La mise en oeuvre du dispositif de distribution d'une matière extrudable conforme à l'invention se déduit aisément de la structure de ses moyens constitutifs qui viennent d'être décrits.

Ce fonctionnement sera maintenant décrit plus en détail en référence aux figures 2 à 4.

Dans une première phase, illustrée par la figure 2, l'embout 7 et le réservoir 6 sont positionnés sur l'embase 1.

A cet effet, l'embout 7 est logé dans le passage 12 de la plaque 11 puis le réservoir 6 est disposé de telle sorte que le piston 2 pénètre dans la chambre 9 et vienne en contact de la matière.

Il est à noter que le piston 2 sert de moyen de support du réservoir 6.

Dans une deuxième phase illustrée par la figure 3, le piston 2 est commandé en déplacement en direction de la plaque 11 au moyen de la poignée 4 ce qui provoque, comme on le comprend, le rapprochement relatif puis la mise en contact de l'embout 7 et du réservoir 6.

A l'issue de cette phase, la face d'étanchéité 14 de la pièce 13 vient au contact de la paroi définissant l'orifice 8 du réservoir.

Dans une troisième phase illustrée par la figure 4, la matière est refoulée vers l'extérieur via l'embout 7.

La force exercée par le piston 2 sur la matière à extruder garantit l'étanchéité entre la surface 14 de la pièce 13 et l'orifice 8.

Comme on le comprend (voir également figure 5), la plaque 11 exerce la réaction à la force du piston en permettant ainsi le raccordement de l'embout 7 au réservoir 6 sans fixation et de manière étanche au moins pendant la phase de refoulement de la matière.

Quelle que soit la force exercée par le piston sur la matière à extruder l'étanchéité est assurée, cette dernière étant d'autant plus forte que la force exercée par le piston est importante.

Pour éviter le retrait du piston pendant la phase de refoulement de la matière, l'embase 1 est de préférence pourvue d'un moyen anti-retour comme par exemple une plaque 17 inclinée en position d'utilisation coopérant avec un ressort et comportant un orifice permettant le libre déplacement de la tige de piston lorsque la plaque est amenée en position droite (c'est-à-dire lorsque ladite plaque est sensiblement orthogonale à la tige de piston) et empêchant le piston 2 de se déplacer dans la direction opposée à celle du déplacement vers la plaque 11 lorsque la plaque 17 est dans sa position inclinée représentée à la figure 1.

Le retrait de l'embout 7 peut être réalisé de façon relativement aisée.

A cet effet, la plaque inclinée 17 est redressée pour permettre le déplacement du piston vers la partie proximale de l'embase. Au cours de cette phase, l'embout 7 peut être facilement séparé du réservoir 6, (dès lors qu'aucune force ne maintient la face d'étanchéité 14 en contact avec la paroi de l'orifice 8 du réservoir 6) et retiré du passage 12 de la plaque 11.

Le dispositif de distribution d'une matière extrudable qui vient d'être décrit présente de très nombreux avantages.

Tout d'abord, il permet une mise en place et un changement aisé de l'embout et/ou du réservoir tout en garantissant une parfaite étanchéité.

Dans le domaine dentaire, il est ainsi possible d'utiliser plusieurs embouts de diamètres différents avec le même réservoir pour des indications différentes, ce qui est impossible avec les dispositifs connus de l'état de la technique qui nécessitent un emmanchement à force de l'embout dans un bouchon, et donc une dimension prédéterminée dudit embout.

Du fait de l'absence de fixation mécanique l'embout et le réservoir peuvent être fabriqués sans problème de tolérance.

L'embase peut être facilement nettoyée et stérilisée avant traitement. L'embout et la pièce d'étanchéité peuvent être avantageusement à usage unique.

Enfin, ce dispositif est d'un coût relativement modéré.

**Revendications**

1. Dispositif de distribution d'une matière extrudable comprenant :

   - un réservoir (6) destiné à contenir la matière et comportant au moins un orifice (8) ;
   - un embout (7) tubulaire séparable communiquant, en position d'utilisation, avec ledit réservoir (6) par ledit orifice (8) ;
   - un piston (2) apte à coulisser, en position d'utilisation, dans ledit réservoir et à exercer sur ladite matière une force suffisante pour la refouler à l'extérieur via l'embout ;
   - une embase (1) portant des moyens de commande en déplacement dudit piston et comportant un élément formant plaque (11) disposé dans un plan sensiblement orthogonal à la direction de déplacement dudit piston (2), ladite plaque (11) présentant un passage (12) apte à recevoir ledit embout (7) et disposé en regard de l'orifice (8) dudit réservoir (6) ;
   - une pièce d'étanchéité (13) disposée entre ledit élément formant plaque 11 et ledit réservoir 6 et présentant un orifice prolongeant le passage de ladite plaque (11) ;
   - des moyens pour réaliser une liaison étanche entre ledit embout (7) et ladite pièce d'étanchéité (13), au moins sous l'effet du déplacement dudit réservoir (6) vers ladite plaque (11) ; et
   - des moyens pour immobiliser ladite pièce d'étanchéité (13) par rapport à ladite plaque (11), au moins sous l'effet du déplacement dudit réservoir (6) vers ladite plaque (11).

   **caractérisé en ce que** ladite pièce d'étanchéité (13) présente une face d'étanchéité (14) tournée vers ledit réservoir (6) ; ladite face d'étanchéité (14) et la paroi définissant l'orifice (8) étant conformées pour coopérer par contact d'appui sensiblement selon une ligne en étant ainsi aptes :

   - d'une part, à coopérer, par contact d'appui, sensiblement sans frottement, sous l'effet du déplacement dudit réservoir (6) vers ladite plaque (11) en réponse au déplacement dudit piston (2) dans ledit réservoir (6), pour réaliser ainsi une liaison étanche ; et
   - d'autre part, à être automatiquement séparées l'une de l'autre sous l'effet du déplacement dudit réservoir en direction opposée à ladite plaque.

2. Dispositif selon la revendication 1, **caractérisé en ce que** ladite pièce d'étanchéité (13) est portée de façon périphérique et coaxiale par ledit embout (7).

3. Dispositif selon la revendication 1 ou 2, **caractérisé en ce que** la pièce d'étanchéité (13) précitée présente une première face (14) sensiblement tronconique formant ladite face d'étanchéité (14) et une deuxième face (15) sensiblement plane apte à venir en appui contre ladite plaque (11) formant ainsi les moyens précités pour immobiliser ladite pièce d'étanchéité par rapport à ladite plaque.

4. Dispositif selon l'une des revendications 1 à 3, **caractérisé en ce que** la face d'étanchéité (14) de la pièce d'étanchéité (13) et la paroi définissant l'orifice (8) du réservoir (6) sont réalisées dans des matières aptes à permettre une déformation relative des surfaces en contact, au moins sous l'effet du déplacement du réservoir (6) vers la plaque (11) en réponse au déplacement du piston (2) dans le réservoir (6).

5. Dispositif selon la revendication 4, **caractérisé en ce que** la face d'étanchéité (14) précitée est réalisée en une matière plastique telle que par exemple du polypropylène.

6. Dispositif selon la revendication 5, **caractérisé en ce que** la paroi de l'orifice (8) du réservoir (6) est réalisée en métal, ou en une matière plastique.

7. Dispositif selon l'une des revendications 1 à 6, **ca-**

**ractérisé en ce que** la paroi définissant l'orifice (8) du réservoir (6) forme un bord chanfreiné.

8. Dispositif selon l'une des revendications 1 à 7, **caractérisé en ce que** l'embase (1) comporte un moyen "anti-retour" destiné à empêcher le déplacement du piston (2) dans une direction opposée à la direction de déplacement vers la plaque (11).

9. Dispositif selon l'une des revendications 1 à 8, **caractérisé en ce que** la pièce d'étanchéité (13) précitée est disposée de telle sorte que l'extrémité proximale de l'embout (7) débouche à l'intérieur de la chambre (9) du réservoir (6) en position d'utilisation.


**Claims**

1. A device for dispensing an extrudable material, the device comprising:

   · a reservoir (6) designed to contain the material and having at least one orifice (8);

   . a separable tubular endpiece (7) communicating, in its in-use position, with said reservoir (6) via said orifice (8);

   · a piston (2) suitable for sliding, in use, inside said reservoir and for exerting sufficient force on said material to cause it to be delivered to the outside via the endpiece;
   · a base (1) carrying means for controlling the displacement of said piston and including a plate-forming element (11) disposed in a plane that is substantially orthogonal to the displacement direction of said piston (2), said plate (11) having a passage (12) suitable for receiving said endpiece (7) and being disposed facing the orifice (8) of said reservoir (6);
   · a sealing piece (13) disposed between said plate-forming element (11) and said reservoir and having an orifice extending the passage through said plate (11);
   · means for making a sealed connection between said endpiece (7) and said sealing piece (13), at least under the effect of said reservoir (6) being displaced towards said plate (11); and
   · means for preventing said sealing piece (13) from moving relative to said plate (11), at least under the effect of the displacement of said reservoir (6) towards said plate (11);

   the device being **characterized in that** said sealing piece (13) has a sealing face (14) facing towards said reservoir (6); said sealing face (14) and the wall defining the orifice (8) being shaped to co-operate by thrust contact substantially along a line thus being suitable:

   · firstly for co-operating by substantially friction-free thrust contact, under the effect of said reservoir (6) being displaced towards said plate (11) in response to the displacement of said piston (2) in said reservoir (6) to establish a sealed connection; and
   · secondly to be automatically separated from each other under the effect of said reservoir being displaced away from said plate.

2. A device according to claim 1, **characterized in that** said sealing piece (13) is carried in peripheral and coaxial manner by said endpiece (7).

3. A device according to claim 1 or 2, **characterized in that** the above-mentioned sealing piece (13) has a substantially frustoconical first face (14) forming said sealing face and a substantially plane second face (14) suitable for being thrust against said plate (11), thus forming the above-mentioned means for preventing said sealing piece from moving relative to said plate.

4. A device according to any one of claims 1 to 3, **characterized in that** the sealing face (14) of the sealing piece (13) and the wall defining the orifice (8) of the reservoir (6) are made of material suitable for enabling relative deformation of the surface that come into contact, at least under the effect of the reservoir (6) being displaced towards the plate (11) in response to the displacement of the piston (2) in the reservoir (6).

5. A device according to claim 4, **characterized in that** the above-mentioned sealing face (14) is made of a plastics material, such as polypropylene, for example.

6. A device according to claim 5, **characterized in that** the wall of the orifice (8) of the reservoir (6) is made of metal, or of a plastics material.

7. A device according to any one of claims 1 to 6, **characterized in that** the wall defining the orifice (8) of the reservoir (6) forms a chamfered edge.

8. A device according to any one of claims 1 to 7, **characterized in that** the base (1) includes "non-return" means designed to prevent the piston (2) being displaced in a direction opposite to its displacement direction towards the plate (11).

9. A device according to any one of claims 1 to 8, **characterized in that** the above-mentioned sealing piece (13) is disposed in such a manner that the proximal end of the endpiece (7) opens up to the inside of the chamber (9) of the reservoir (6) in the in-use position.

**Patentansprüche**

1. Verteilungsvorrichtung für ein extrudierbares Material, umfassend:

   - einen Behälter (6), der dazu bestimmt ist, das Material zu enthalten, und der mindestens eine Öffnung (8) umfasst;
   - einen abtrennbaren röhrenförmigen Ansatz (7), der in Verwendungsposition mit dem Behälter (6) durch die Öffnung (8) in Verbindung steht;
   - einen Kolben (2), der in Verwendungsposition in dem Behälter gleiten und auf das Material eine ausreichend starke Kraft ausüben kann, um es über den Ansatz nach außen zu befördern;
   - einen Sockel (1), der Steuermittel für die Verschiebung des Kolbens trägt und ein eine Platte bildendes Element (11) umfasst, das in einer im Wesentlichen zur Verschieberichtung des Kolbens (2) orthogonalen Ebene angeordnet ist, wobei die Platte (11) einen Durchgang (12) aufweist, der den Ansatz (7) aufnehmen kann und gegenüber der Öffnung (8) des Behälters (6) angeordnet ist;
   - ein Dichtungsteil (13), das zwischen dem die Platte (11) bildenden Element und dem Behälter (6) angeordnet ist und eine Öffnung aufweist, die den Durchgang der Platte (11) verlängert;
   - Mittel, um eine dichte Verbindung zwischen dem Ansatz (7) und dem Dichtungsteil (13) zumindest unter der Wirkung der Verschiebung des Behälters (6) zu der Platte (11) herzustellen; und
   - Mittel, um das Dichtungsteil (13) in Bezug auf die Platte (11) zumindest unter der Wirkung der Verschiebung des Behälters (6) zu der Platte (11) festzustellen,

   **dadurch gekennzeichnet, dass** das Dichtungsteil (13) eine Dichtungsfläche (14) aufweist, die zu dem Behälter (6) gewandt ist; wobei die Dichtungsfläche (14) und die Wand, die die Öffnung (8) definiert, derart ausgebildet sind, dass sie durch Stützkontakt im Wesentlichen entlang einer Linie zusammenwirken und somit in der Lage sind:

   - einerseits durch Stützkontakt im Wesentlichen ohne Reibung unter der Wirkung der Verschiebung des Behälters (6) zu der Platte (11) als Reaktion auf die Verschiebung des Kolbens (2) in dem Behälter (6) zusammenzuwirken, um so eine dichte Verbindung herzustellen; und

   - andererseits automatisch voneinander unter der Wirkung der Verschiebung des Behälters in die zu der Platte entgegengesetzte Richtung getrennt zu werden.

2. Vorrichtung nach Anspruch 1, **dadurch gekennzeichnet, dass** das Dichtungsteil (13) von dem Ansatz (7) in peripherer und koaxialer Weise getragen wird.

3. Vorrichtung nach Anspruch 1 oder 2, **dadurch gekennzeichnet, dass** das vorgenannte Dichtungsteil (13) eine erste im Wesentlichen kegelstumpfartige Fläche (14), die die Dichtungsfläche (14) bildet, und eine zweite im Wesentlichen ebene Fläche (15) aufweist, die an der Platte (11) zur Abstützung gelangen kann und so die vorgenannten Mittel zur Feststellung des Dichtungsteils in Bezug auf die Platte bildet.

4. Vorrichtung nach einem der Ansprüche 1 bis 3, **dadurch gekennzeichnet, dass** die Dichtungsfläche (14) des Dichtungsteils (13) und die die Öffnung (8) des Behälters (6) definierende Wand aus Materialien hergestellt sind, die eine relative Verformung der in Kontakt befindlichen Flächen zumindest unter der Wirkung der Verschiebung des Behälters (6) zu der Platte (11) als Reaktion auf die Verschiebung des Kolbens (2) in dem Behälter (6) ermöglichen können.

5. Vorrichtung nach Anspruch 4, **dadurch gekennzeichnet, dass** die vorgenannte Dichtungsfläche (14) aus einem Kunststoff, wie beispielsweise Polypropylen, hergestellt ist.

6. Vorrichtung nach Anspruch 5, **dadurch gekennzeichnet, dass** die Wand der Öffnung (8) des Behälters (6) aus Metall oder aus einem Kunststoff hergestellt ist.

7. Vorrichtung nach einem der Ansprüche 1 bis 6, **dadurch gekennzeichnet, dass** die die Öffnung (8) des Behälters (6) bildende Wand eine abgeschrägte Kante bildet.

8. Vorrichtung nach einem der Ansprüche 1 bis 7, **dadurch gekennzeichnet, dass** der Sockel (1) ein "Rückkehrverhinderungsmittel" umfasst, das dazu bestimmt ist, die Verschiebung des Kolbens (2) in eine zu der Verschieberichtung zur Platte (11) entgegengesetzte Richtung zu verhindern.

9. Vorrichtung nach einem der Ansprüche 1 bis 8, **dadurch gekennzeichnet, dass** das vorgenannte Dichtungsteil (13) derart angeordnet ist, dass das proximale Ende des Ansatzes (7) im Inneren der Kammer (9) des Behälters (6) in Verwendungsposition mündet.

FIG.1

EP 0 944 440 B1

FIG.2

FIG.3

FIG.4

EP 0 944 440 B1

FIG.5